# EUROPÄISCHE PATENTSCHRIFT

(11) **EP 2 391 382 B1**
(45) Veröffentlichungstag und Bekanntmachung des Hinweises auf die Patenterteilung: **04.06.2014**
(21) Anmeldenummer: 09776346.0
(22) Anmeldetag: 29.01.2009
(51) Int. Cl.: A61K 38/46, A61P 1/18

(54) **PHARMAZEUTISCHES PRÄPARAT ENTHALTEND LIPASE BAKTERIELLEN URSPRUNGS**
PHARMACEUTICAL PREPARATION COMPRISING LIPASE OF BACTERIAL ORIGIN
PRÉPARATION PHARMACEUTIQUE CONTENANT DES LIPASES D'ORIGINE BACTÉRIENNE

(43) Veröffentlichungstag der Anmeldung: 07.12.2011
(73) Patentinhaber: Nordmark Arzneimittel GmbH & Co. KG, 25436 Uetersen (DE)
(72) Erfinder: KURFÜRST, Manfred, 25436 Moorrege (DE); FRIEDEL, Rainer, 30161 Hannover (DE); FRIEDRICH, Olaf, 25436 Tornesch (DE); HÜTTLER, Silke, 25335 Altenmoor (DE); RÄMSCH, Christian, 25436 Gross Nordende (DE)
(74) Vertreter: Richter Werdermann Gerbaulet Hofmann
(86) Internationale Anmeldenummer: PCT/EP2009/000566
(87) Internationale Veröffentlichungsnummer: WO 2010/085975

(56) Entgegenhaltungen:
- WO-A1-2006/044529
- WO-A1-2008/127567
- US-A1- 2005 250 817

## Beschreibung

### Zusammenfassung der Erfindung

Die Erfindung betrifft pharmazeutische Präparate zur Behandlung von Pankreasinsuffizienz, beispielsweise Mukoviszidose oder anderer Pankreaserkrankungen. Insbesondere betrifft die vorliegende Erfindung flüssige pharmazeutische Präparate von Verdauungsenzymen, die keinen Überzug haben und in der aggressiven Umgebung von Magen/Zwölffingerdarm aktiv sind, d.h. die Erfindung betrifft ein pharmazeutisches Präparat, umfassend Bakterien-Lipase in wässriger Lösung. Außerdem sind diese flüssigen pharmazeutischen Präparate stabil und können bei hohen Temperaturen gelagert werden.

### Hintergrund der Erfindung

Übermäßige Expression oder Fehlen wirksamer Enzyme führen häufig zu Stoffwechsel- oder Magendarmerkrankungen. Unausgeglichenheit der Lipasespiegel kann beispielsweise eine Vielzahl an Verdauungsstörungen verursachen, inklusive Malabsorption von Fetten. Bei Patienten, die unter Mukoviszidose, chronischer Pankreatitis und anderen Pankreaserkrankungen leiden, tritt Malabsorption von Fetten auf. Gewöhnlich beobachtete Folgen der Malabsorption von Fetten sind Unterleibsbeschwerden, Steatorrhoea (Fettdurchfall), Mangel an essentiellen Fettsäuren, an fettlöslichen Vitaminen (beispielsweise A, D, E und K) und allgemeine Entwicklungsstörung.

Das übliche Vorgehen zur Behandlung von Erkrankungen mit Lipaseinsuffizienz sind oral verabreichte Lipaseenzyme, meistens Produkte auf der Basis von Schweinepankreas, das entfettet, getrocknet und zerstoßen wurde. Das entstehende Produkt besteht aus mehreren Enzymen, wie Lipase, Amylasen, Proteasen, Esterasen etc.

Allgemein verfügbare Verabreichungsformen sind üblicherweise mit magensaftresistentem Film überzogene Tabletten, Mikrotabletten, Mikrodragees, Kapseln, Pulver und Granulate. Der Nachteil dieser Verabreichungsformen besteht in der Unannehmlichkeit der Verabreichung an den Patienten, der große Tabletten oder Kapseln schlucken muss. Insbesondere im Fall von Patienten, die nicht in der Lage sind, Tabletten zu schlucken, wie kleine Kinder, oder die künstliche Ernährung brauchen, sind diese Verabreichungsformen nur schwer anwendbar. Das Zerstoßen der Tabletten kann keine homogene Verteilung der Enzyme im Chymus sicherstellen und die geringe Löslichkeit des Produktes kann zur Verstopfung der Ernährungsrohre führen.

Flüssige Formulierungen von Verdauungsenzymen wurden beispielsweise in EP 0826375 oder US 2006/0128587 beschrieben. In diesen Patentanmeldungen werden die Enzyme entweder mit Zusätzen stabilisiert oder durch Modifizierung der Enzyme selbst. In beiden Fällen jedoch müssen die Enzymlösungen frisch vor der Verabreichung zubereitet werden, was weniger bequem ist, als ein gebrauchsfertiges flüssiges Präparat. Außerdem müssen die Wirkungen von Zusätzen auf den Wirkstoff, die Wirksamkeit und die Sicherheit in Betracht gezogen werden.

Die Behandlung auf der Grundlage leicht verfügbarer Pankreatin-Produkte hat verschiedene Nachteile. Die Lipase wird als das Leitenzym angesehen. Aufgrund der niedrigen spezifischen Lipase-Aktivität muss eine Menge von bis zu 5-10 g pro Tag von den Patienten eingenommen werden. Außerdem ist die Schweine-Lipase in einem pH-Bereich von 5 bis 9 aktiv und ist daher während des Magendurchganges inaktiv.

Außerdem muss die Lipase-Aktivität aufgrund der Empfindlichkeit auf niedrigen pH-Wert vor den sauren pH-Bedingungen im Magen geschützt werden, beispielsweise durch magensaftresistenten Überzug der Tabletten. In einigen medizinischen Anwendungen sind die begleitenden proteolytischen oder amylolytischen Enzymaktivitäten unerwünscht: Der Amylase-Gehalt ist bei Kindern mit Mukoviszidose unerwünscht, während Lipasen therapeutisch erforderlich sind. Proteasen sind bei Patienten mit akuter Pankreatitis oder aktiven Phasen chronischer Pankreatitits kontraindiziert (siehe US 5 645 832). Deshalb ist die Verfügbarkeit einer Lipase als einzelnes Protein vorteilhaft. In US 5 645 832 oder US 5 489 530 wurde bereits eine aus Bakterien gewonnene Lipase im Einzelnen beschrieben. Außer der Bereitstellung eines gereinigten einzelnen Proteins, ist die Lipase-Konzentration von Bakterien-Lipase sehr hoch, so dass nur geringe Substanzmengen (ungefähr 0,2 g) verabreicht werden müssen. Der pH-Bereich für die Aktivität der Bakterien-Lipasen liegt zwischen 3 und 9 und überwindet daher die Einschränkungen der Stabilität und Aktivität von Schweine-Lipase/-Pankreatin. Dies bedeutet, dass die lipolytische Wirkung der Bakterien-Lipasen mit größerer Wirkung im Magen-DarmTrakt angewendet werden kann, als die für die Therapie von Maldigestion auf dem Markt geläufigen Produkte. Zusätzlich ist die bakterielle Lipase ein tierfreies Produkt, das die Gefahr möglicher, aus tierischen Quellen stammender Virus-Verseuchung vermeidet.

Die US 2005/0250817 A1 offenbart eine pharmazeutische Zusammensetzung zur oralen Verabreichung, die bei Kontakt mit einer hydrophilen und einer lipophilen Phase sich selbstemulgierend verhält, wobei die Zusammensetzung (i) ein Enzym oder eine Enzymmischung mit zumindest lipolytischer Aktivität und (ii) ein System mit mindestens einem oberflächen aktiven Mittel bzw. Tensid und mindestens ein Co-Tensid aufweist.

Die WO 2008/127567 A1 offenbart eine Nährstoff- bzw. Ernährungsproduktzusammensetzung, umfassend ein Enzym, ausgewählt aus der Gruppe, bestehend aus einer Lipase, Amylase, Protease und einer Kombination dieser, wobei das Enzym derart formuliert ist, dass in wässrigem Medium, wie beispielsweise der Speichelflüssigkeit, eine nachhaltige Stabilität vorliegt. Nährstoff- bzw. Ernährungsproduktzusammensetzung weist zudem einen Ernährungszusatz auf.

Die WO 2006/044529 A1 beschreibt eine Zusammensetzung, umfassend Lipase, Protease und Amylase, wobei das Verhältnis Lipase, Protease und Amylase in der Zusammensetzung etwa 1:1:0,15 UPS-Einheiten beträgt. Die angegebenen oralen Dosierungsformen sind Tabletten, Kapseln, Aufschlämmungen ("slurries"), Portionsbeutel, Suspensionen und Dragees. Lösungen, insbesondere wässrige Lösungen, der Enzymmischung werden nicht beschrieben.

### Beschreibung der Erfindung

Die vorliegende Erfindung schafft ein flüssiges pharmazeutisches Präparat, das ein Verdauungsenzym in Form von Bakterien-Lipase in wässriger Lösung für die Behandlung von Verdauungsstörungen enthält. Das Präparat dient der Behandlung von Patienten mit Pankreasinsuffizienz, beispielsweise Mukoviszidose oder anderen Pankreaserkrankungen.

Überraschenderweise konnte gezeigt werden, dass Lipase ohne Überzug in der aggressiven Umgebung von Magen/Zwölffingerdarm aktiv ist und dass das Enzym in wässriger Lösung über mehrere Monate bei Raumtemperatur stabil ist. Zusätzlich wurde festgestellt, dass Bakterien-Lipase in der Lage ist, Fettdurchfall bei Mukoviszidose-Patienten zu verringern.

Die Erfindung beruht auf einer Lipase, die in Bakterien erzeugt wird, und ihre Herstellung wird in US 5 645 832 ausdrücklich beschrieben.

Die Lipase-Anwendung wurde als wirksame Behandlung von Patienten nachgewiesen, die unter Pankreatitis oder Mukoviszidose leiden. Die Verringerung von Fettdurchfall wurde bei ausschließlicher Anwendung von Lipase beobachtet. Keine anderen Enzyme, wie Proteasen, Amylasen oder Esterasen waren anwesend (Beispiel 1).

Reine Bakterien-Lipase kann spezifische Aktivitäten von mehr als 3,5 Milliarden U/g aufweisen. Aufgrund dieser hohen lipolytischen Aktivität muss den Patienten eine geringere Menge Verabreichungsform (d. h. Anzahl an Tabletten oder Volumen an Lösung) verabreicht werden.

Die vorliegende Erfindung bietet die Lipase als flüssiges pharmazeutisches Präparat an, um die Einschränkungen der geläufigen Formulierungen (Tabletten) therapeutischer Verdauungsenzyme zu überwinden. Insbesondere im Fall von Patienten, die nicht in der Lage sind, Tabletten zu schlucken, wie kleine Kinder, oder solche, die künstliche Ernährung brauchen, sind die geläufigen Verabreichungsformen nicht oder nur bedingt anwendbar.

Ein flüssiges pharmazeutisches Produkt bietet die Möglichkeit einer bequemen Dosierung des Medikaments, homogene Verteilung des Enzyms in der Nahrung und Anwendung bei künstlicher Ernährung.

Es konnte gezeigt werden, dass Lipase ohne Überzug unter pH-Bedingungen aktiv ist, die die aggressive Umgebung des Magens bei der Einnahme imitiert (Beispiel 2). Außerdem wurde gezeigt, dass die Bakterien-Lipase in wässriger Lösung für mindestens 3 Monate sogar bei hohen Temperaturen stabil ist (Beispiel 3). Keine chemische Modifizierung, wie sie in US 2006/0128587 beschrieben wird, war erforderlich.

In der Erfindung eingeschlossen sind Formulierungen, die außerdem Stabilisatoren verwenden, wie
- Salze
- organische Säuren
- Aminosäuren
- Detergentien
- Zucker
- Öle
- Viskositätsregulierungsmittel.

Bei der flüssigen Formulierung handelt es sich um eine wässrige Lösung. Die Erfindung beruht auf den Vorteilen flüssiger Verabreichungsformen insbesondere, aber nicht ausschließlich, für Kinder.

Flüssige Verabreichungsformen können entweder dadurch bereitet werden, dass die Lipase direkt als flüssige Lösung hergestellt wird, oder durch Lösen des aktiven Bestandteils Lipase in einem wässrigen Lösungsmittel.

Verabreichungsformen in Form von wässrigen Lösungen von Lipase sind aus einer Reihe von Gründen nützlich. Sie können für verschiedene Verabreichungswege formuliert werden: Orale Einnahme, Einführen in Körperhohlräume oder äußere Anwendung. Die Dosis kann durch Verdünnung eingestellt werden und einzelne Tropfen können beispielsweise mit einer Tropfvorrichtung verabreicht werden. Die flüssige orale Form kann leicht Kindern oder Menschen verabreicht werden, die unfähig sind, Tabletten, Kapseln oder beliebige andere feste Verabreichungsformen zu schlucken.

Die Lipaselösung ist ein homogenes Gemisch, das pharmazeutische Formen einschließt, die als Wasser, aromatische Wässer, wässrige Säuren, Lösungen bezeichnet werden.

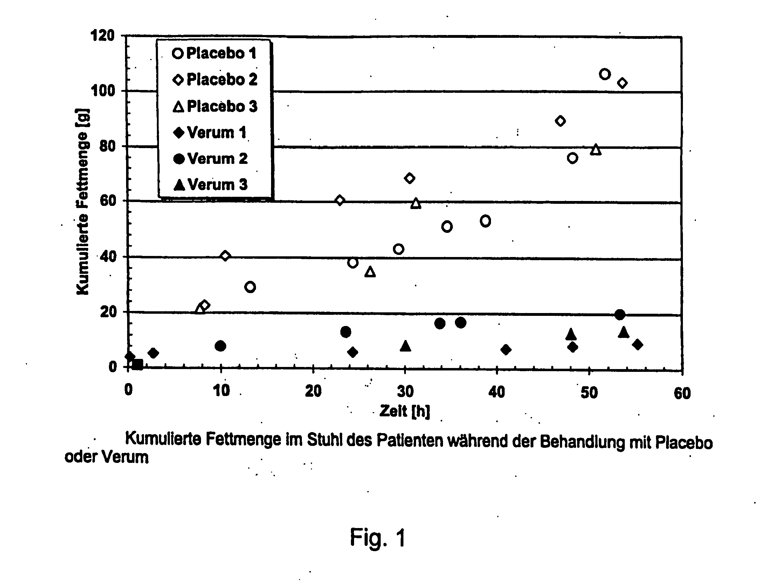

### Beispiel 1: Kumulierte Fettmenge im Stuhl des Patienten während der Behandlung mit Placebo oder Verum - Fig. 1 -

Bakterielle Lipase wurde während einer klinischen Studie mit mukoviszidose Patienten getestet. Wie in Fig. 1 und der nachstehenden Tabelle mit der Angabe des Fettadsorptionskoeffizienten von Patienten unter Placebo und Verum gezeigt, sind signifikante Unterschiede in der Fettmenge im Stuhl der Patienten unter Verum als auch der Fettadsorptionskoeffizient (CFA) zu sehen. Die bakterielle Lipase verbessert signifikant den Abbau von Fett während der Verdauung.

| **Patient** | **CFA** |
|---|---|
| Placebo 1 | 73 % |
| Placebo 2 | 64 % |
| Placebo 3 | 65 % |
| Verum 1 | 92 % |
| Verum 2 | 96 % |
| Verum 3 | 93 % |

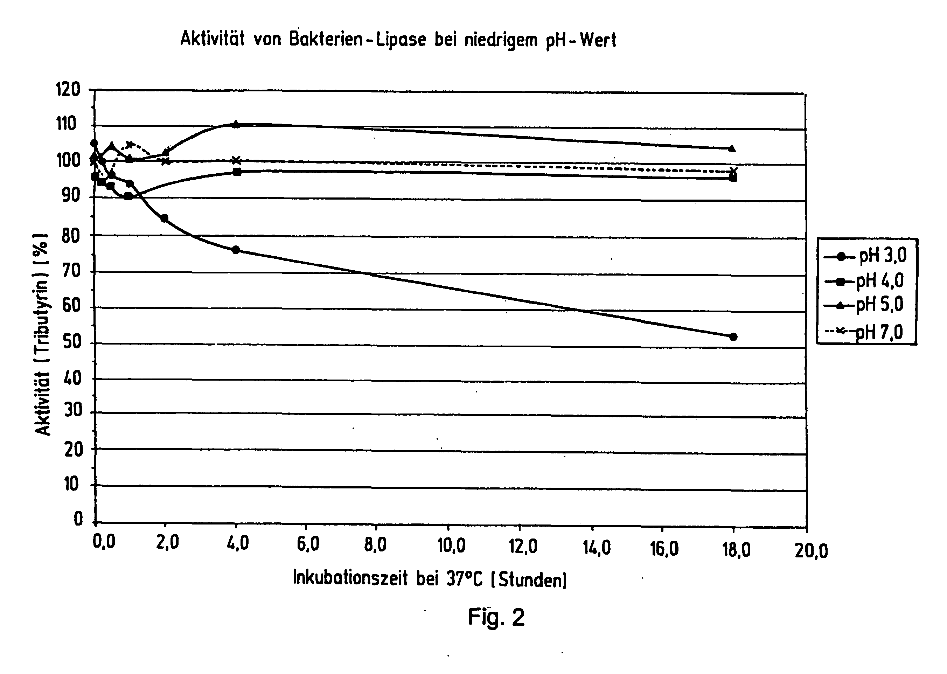

### Beispiel 2: Aktivität von Lipase unter Bedingungen niedrigen pH-Wertes (Imitation des pH-Wertes im Magen) - Fig. 2 -

Bakterien-Lipase wurde gelöst und auf den pH-Wert von 1,0, 2,0, 3,0, 4,0 und 5,0 eingestellt. Lipaseaktivitäten wurden unter Anwendung des Tributyrin-Tests nach 15, 30 min und 1, 2, 4 und 18 Stunden beim Test-pH-Wert (8,0) gemessen.

Es wurde festgestellt, dass der pH-Wert im Magen von Patienten, die unter Mukoviszidose leiden, beim Schlucken auf pH 4 ansteigt. Bakterien-Lipase zeigt unter diesen Bedingungen eine ausreichende Aktivität (Fig. 2), während die Aktivität von Schweine-Lipase (aus Pankreatin) nach 2 Stunden um 85% verringert ist und nach 4 Stunden bei pH 4 um 96% (nicht dargestellt).

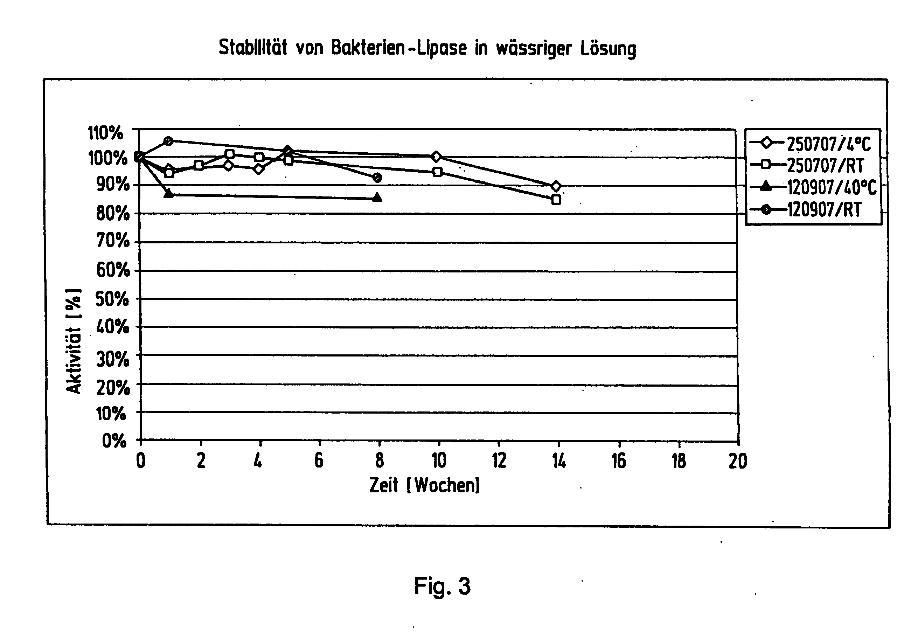

### Beispiel 3: Stabilität von Bakterien-Lipase in wässriger Lösung - Fig. 3 -

Bakterien-Lipase wurde in Wasser gelöst und bei 4°C, Raumtemperatur und 40°C inkubiert. Die Aktivität wurde nach 1, 2, 3, 4, 5, 10 und 14 Wochen gemessen. Kein erheblicher Aktivitätsverlust wurde in diesem Zeitraum festgestellt.

Die Erfindung umfasst die Beschreibung mit den Beispielen, die Ansprüche und die Abbildungen.

## Patentansprüche

1. Pharmazeutisches Präparat, umfassend
Bakterien-Lipase in wässriger Lösung für die Behandlung von Verdauungsstörungen.

2. Pharmazeutisches Präparat für die Behandlung nach Anspruch 1, wobei die Verdauungsstörung eine Pankreas-Erkrankung ist
- Mukoviszidose
- Pankreatitis.

3. Pharmazeutisches Präparat für die Behandlung nach einem der vorhergehenden Ansprüche 1 oder 2, **dadurch gekennzeichnet, dass** die wässrige Lösung zubereitet wird durch
a) direkte Herstellung der Lipase als flüssige Lösung, oder
b) Lösung des aktiven Bestandteils Lipase in einem wässrigen Lösungsmittel und bevorzugter Weise bei 4°C, Raumtemperatur und 40°C inkubieren.

4. Pharmazeutisches Präparat für die Behandlung nach einem der vorhergehenden Ansprüchen 1 bis 3, wobei die Bakterien-Lipase ist
- von der Art Burkholderia
- von der Art Pseudomonas
- von Burkholderia plantarii.

5. Flüssiges und stabiles pharmazeutisches Präparat für die Behandlung nach einem der Ansprüche 1 bis 4 zur Verwendung durch Anwendung von Lipase in Form einer stabilen Lösung zur Behandlung von Pankreasinsuffizienz, wie Mukoviszidose oder andere Pankreaserkrankungen.

6. Lipase als stabile Lösung in flüssiger Form mit Stabilisatoren, wie Salzen, organischen Säuren, Aminosäuren, Detergentien, Zucker, zur Verwendung zur Behandlung von Pankreasinsuffizienz, wie Mukoviszidose oder andere Pankreaserkrankungen.

## Claims

1. A pharmaceutical preparation comprising bacterial lipase in aqueous solution for the treatment of digestion disorders.

2. The pharmaceutical preparation for the treatment according to Claim 1, wherein the digestion disorder is a pancreatic illness:
- mucoviscidosis
- pancreatitis.

3. The pharmaceutical preparation for the treatment according to one of preceding Claims 1 or 2, **characterized in that** the aqueous solution is prepared by
a) directly producing the lipase as a liquid solution or
b) dissolving the active constituent lipase in an aqueous solvent and preferably incubating at 4 °C, room temperature, or 40 °C.

4. The pharmaceutical preparation for the treatment according to one of preceding Claims 1 to 3, wherein the bacterial lipase is
- of the Burkholderia type,
- of the Pseudomonas type,
- of the Burkholderia plantarii type.

5. A liquid and stable pharmaceutical preparation for the treatment according to one of Claims 1 to 4, for use by applying lipase in the form of a stable solution for the treatment of pancreatic insufficiency, such as mucoviscidosis, or other pancreatic illnesses.

6. A lipase as a stable solution in liquid form comprising stabilizers, such as salts, organic acids, amino acids, detergents and sugar, for use for the treatment of pancreatic insufficiency, such as mucoviscidosis, or other pancreatic illnesses.

## Revendications

1. Préparation pharmaceutique, comprenant
une lipase bactérienne en solution aqueuse pour le traitement de troubles digestifs.

2. Préparation pharmaceutique de traitement selon la revendication 1, le trouble digestif étant un trouble pancréatique
- mucoviscidose
- pancréatite.

3. Préparation pharmaceutique de traitement selon l'une quelconque des revendications 1 ou 2 précédentes, **caractérisé en ce qu'**on prépare la solution aqueuse par
a) une production directe de la lipase en tant que solution aqueuse
ou
b) une dissolution du principe actif lipase dans un solvant aqueux et de préférence à une température ambiante de 4°C et avec une incubation à 40°C.

4. Préparation pharmaceutique de traitement selon l'une quelconque des revendications 1 à 3 précédentes, la lipase bactérienne étant
- du genre Burkholderia
- du genre Pseudomonas
- du Burkholderia plantarii.

5. Préparation pharmaceutique liquide et stable de traitement selon l'une quelconque des revendications 1 à 4 précédentes destinée à être utilisée par mise en oeuvre d'une lipase sous la forme d'une solution stable pour le traitement d'insuffisance pancréatique, comme la mucoviscidose ou d'autres troubles pancréatiques.

6. Lipase en tant que solution stable sous forme liquide avec des stabilisateurs, comme des sels, des acides organiques, des acides aminés, des détergents, du sucre, destinée à être utilisée pour le traitement d'insuffisance pancréatique, comme la mucoviscidose ou d'autres troubles pancréatiques.
